# EUROPÄISCHE PATENTSCHRIFT

(11) **EP 4 527 330 B1**
(45) Veröffentlichungstag und Bekanntmachung des Hinweises auf die Patenterteilung: **22.04.2026**
(21) Anmeldenummer: 24200985.0
(22) Anmeldetag: 18.09.2024
(51) Int. Cl.: A61B 18/14, A61B 17/34

(54) **SCHLITTENVORRICHTUNG FÜR EIN CHIRURGISCHES INSTRUMENT**
CARRIAGE ASSEMBLY FOR A SURGICAL INSTRUMENT
DISPOSITIF DE COULISSEMENT POUR INSTRUMENT CHIRURGICAL

(30) Priorität: 21.09.2023 DE 102023125617
(43) Veröffentlichungstag der Anmeldung: 26.03.2025
(73) Patentinhaber: Karl Storz SE & Co. KG, 78532 Tuttlingen (DE)
(72) Erfinder: WITTKE, Uwe, 78532 Tuttlingen (DE)

(56) Entgegenhaltungen:
- DE-A1- 102015 016 099
- DE-B3- 102013 018 972
- US-B2- 9 474 438

## Beschreibung

### GEBIET DER ERFINDUNG

Die vorliegende Erfindung betrifft eine Schlittenvorrichtung für ein chirurgisches Instrument mit zumindest zwei Instrumenten sowie ein chirurgisches Instrument mit zwei Instrumenten, aufweisend eine Schlittenvorrichtung.

### TECHNISCHER HINTERGRUND

Chirurgische Instrumente werden für unterschiedliche Anwendungen eingesetzt. Beispielsweise können diese im Bereich minimalinvasiver Operationen verwendet werden und über ein chirurgisches Werkzeug verfügen. Chirurgische Instrumente können als Resektoskop ausgebildet sein und über ein Arbeitselement verfügen, das beispielsweise zur Führung des chirurgischen Werkzeugs, wie einer Elektrode, ausgebildet ist.

Resektoskope sind insbesondere für urologische oder beispielsweise gynäkologische Anwendungen bekannt. Dabei kann das Arbeitselement mit der Elektrode durch die Harnröhre bis zu einem Operationsgebiet in den Körper eines Patienten eingeführt werden. An einem distalen Ende (d.h. benutzerfern) weist die Elektrode beispielsweise eine Schneidschlinge auf, mittels welcher Gewebe abgetragen werden kann. Das abgetragene Gewebe kann mittels einer Spülflüssigkeit, die durch einen Spülkanal bzw. Zulaufkanal zum Operationsgebiet geführt wird, durch einen Ablaufkanal wieder abgeführt werden. Der Zulauf- und der Ablaufkanal ermöglichen eine kontinuierliche Spülung, die zugleich zum Freihalten eines Sichtfensters dient, um stets eine einwandfreie endoskopische Sicht zu ermöglichen.

Chirurgische Instrumente weisen allgemein eine Handhabe mit einem Schlitten auf, der entlang einer Längsachse des chirurgischen Instruments verschiebbar gelagert und mit dem chirurgischen Werkzeug verbunden ist. Wenn ein Instrument, wie eine Elektrode, in das Arbeitselement eingesetzt ist, kann durch axiales Bewegen des Schlittens die Schneidschlinge der Elektrode in axialer Richtung verschoben werden, um beispielsweise Gewebe abzutragen.

In das chirurgische Instrument können unterschiedliche Instrumente, wie beispielsweise Elektroden, Laserfasern oder ähnliches, eingesetzt sein. Um das Instrument in dem chirurgischen Instrument zu verwenden, muss der Schlitten an das Instrument gekoppelt werden, sodass das Instrument durch die Bewegung des Schlittens in dem chirurgischen Instrument bewegt werden kann.

Durch die Verwendung unterschiedlicher Instrumente in einem chirurgischen Instrument, wodurch ein Ein- und Ausbau der unterschiedlichen Instrumente erforderlich ist, wird Zeit beansprucht.

US 9 474 438 B2 offenbart ein Endoskop mit einem Arbeitselement, das so ausgebildet ist, dass es mindestens ein erstes Instrument und ein zweites Instrument trägt. Das Arbeitselement kann mindestens eine entlang der Rotationsachse verlaufende Führungsschiene aufweisen, und ein Betätigungsblock kann verschiebbar an der mindestens einen Führungsschiene montierbar und an mindestens einem der Instrumente befestigbar sein, um das mindestens eine der inneren Instrumente in Längsrichtung der Außenschutzhülle bewegen zu können.

DE 10 2015 016099 A1 offenbart ein Resektoskop mit einer Elektrode, die mit ihrem proximalen Endbereich an einem Kopplungskörper mit einem an diesem endenden Kabel elektrisch kontaktiert ist, wobei der Kopplungskörper in einem am Resektoskop längsverschiebbaren Schlitten abnehmbar angeordnet ist und wobei die Elektrode mittels eines Arretierelementes arretierbar ist, wobei die Elektrode am Kopplungskörper arretierbar ist.

### ZUSAMMENFASSUNG DER ERFINDUNG

Vor diesem Hintergrund liegt der vorliegenden Erfindung die Aufgabe zugrunde, eine Verwendung von zumindest zwei Instrumenten in einem chirurgischen Instrument zu optimieren. Erfindungsgemäß wird diese Aufgabe durch eine Schlittenvorrichtung mit den Merkmalen des Patentanspruchs 1 und/oder durch ein chirurgisches Instrument mit den Merkmalen des Patentanspruchs 17 gelöst.

Demgemäß ist vorgesehen:
Eine Schlittenvorrichtung für ein chirurgisches Instrument mit zumindest zwei Instrumenten, mit einer Schlittenbasis, einer zur Betätigung ausgebildeten Kinematikeinheit, mit einem ersten Schlittenmodul, das zur Kopplung mit einem ersten Instrument ausgebildet ist,
und mit einem zweiten Schlittenmodul, das zur Kopplung mit einem zweiten Instrument ausgebildet ist, wobei die beiden Schlittenmodule relativ zueinander verschiebbar und einzeln mit der Kinematikeinheit koppelbar sind, um das erste Instrument oder das zweite Instrument zu verwenden, und wobei das ersten Schlittenmodul und das zweite Schlittenmodul an der Schlittenbasis fixierbar sind, wenn diese nicht in Verwendung sind.
Ein chirurgisches Instrument mit zwei Instrumenten, aufweisend eine Schlittenvorrichtung. Die Instrumente sind über die Schlittenmodule unabhängig voneinander mit der Kinematikeinheit koppelbar.

Die der vorliegenden Erfindung zugrundeliegende Erkenntnis besteht darin, dass ein Schlitten mit unterschiedlichen Instrumenten eines chirurgischen Instruments koppelbar ist, ohne die Instrumente ein- oder auszubauen.

Die der vorliegenden Erfindung zugrundeliegende Idee besteht darin, den Schlitten mehrteilig auszubilden, wodurch zumindest zwei Schlittenmodule bereitgestellt werden. Jedes dieser Schlittenmodule ist mit einem Instrument gekoppelt. Soll ein Instrument verwendet werden, so wird das entsprechende Schlittenmodul mit einer Kinematikeinheit des chirurgischen Instruments gekoppelt. Das Instrument kann dann wie gewöhnlich eingesetzt werden. Soll das andere Instrument verwendet werden, so kann das andere Schlittenmodul mit der Kinematikeinheit gekoppelt werden. Es wird demnach ein modularer, insbesondere geteilter, Schlittenaufbau vorgeschlagen.

Mit anderen Worten können die Instrumente unabhängig voneinander bzw. selektiv mit der Kinematikeinheit gekoppelt werden. Das Schlittenmodul kann dabei auch für mehr als zwei unterschiedliche Instrumente ausgebildet bzw. mehrfach geteilt sein, wobei jedes der Instrumente unabhängig mit der Kinematikeinheit gekoppelt werden kann. So können auch mehr als zwei Schlittenmodule vorgesehen sein, die jeweils wie beschrieben ausgebildet sein können.

Die Kinematikeinheit kann beispielsweise einen Teil des Schlittens selbst ausbilden. Des Weiteren kann an der Kinematikeinheit ein Daumenring, eine Handhabe oder ähnliches angeordnet sein, sodass das entsprechende Schlittenmodul hin- und her bewegt werden kann.

Die Kinematikeinheit ist zur Betätigung ausgebildet. Dies bedeutet insbesondere, dass die Kinematikeinheit entlang einer Längsrichtung des chirurgischen Instruments, welche insbesondere eine vorbestimmte Achse ausbildet, bewegt werden kann. Durch die Kinematikeinheit kann daher insbesondere die Bewegung der Instrumente gesteuert werden.

Die Schlittenbasis kann insbesondere an einer zu einem distalen Ende des chirurgischen Instruments gegenüberliegenden Seite an der Schlittenvorrichtung angeordnet sein. Bevorzugt ist die Schlittenbasis an einer Seitenfläche der Schlittenvorrichtung angeordnet. Insbesondere kann die Schlittenbasis eine Seitenfläche der Schlittenvorrichtung ausbilden. Die Schlittenbasis kann als flächiges Element, insbesondere als Plattenelement, ausgebildet sein.

Die Kinematikeinheit kann angrenzend zu der Schlittenbasis angeordnet sein. Die Kinematikeinheit kann einen Teil eines Gehäuses, einen Daumenring oder weitere Elemente umfassen, die bei Verwendung eines Instruments entlang einer Längsachse des chirurgischen Instruments verschoben werden. Bevorzugt kann die Kinematikeinheit von der Schlittenbasis wegbewegt werden, wobei durch die Kinematikeinheit auch die Schlittenmodule von der Schlittenbasis wegbewegt werden können. Damit kann das jeweils gewählte Instrument verwendet werden.

Die Schlittenbasis ist insbesondere als ortsfeste Schlittenbasis ausgebildet. Dies bedeutet insbesondere, dass die Schlittenbasis ortsfest ausgebildet ist, während sich die Kinematikeinheit relativ zu der Schlittenbasis bewegen bzw. verschieben kann. Da die Schlittenmodule mit der Kinematikeinheit gekoppelt werden können, können die Schlittenmodule bezüglich der ortsfesten Schlittenbasis verschoben bzw. bewegt werden.

Das erste Schlittenmodul ist zur Kopplung mit einem ersten Instrument ausgebildet. Dies bedeutet insbesondere, dass das erste Instrument an dem ersten Schlittenmodul fixiert werden kann. Dies kann beispielsweise über zumindest eine Führungshülse erfolgen, die an dem ersten Schlittenmodul fixiert ist und in welcher das erste Instrument geführt werden kann. Alternativ oder zusätzlich können Befestigungsmittel vorgesehen sein, mit welchen das erste Instrument mit dem ersten Schlittenmodul befestigt ist. Eine Bewegung des ersten Schlittenmoduls kann daher direkt auf das erste Instrument übertragen werden. Die gemachten Darstellungen gelten ebenso für das zweite Schlittenmodul mit dem zweiten Instrument.

Bei den Instrumenten kann es sich um Elektroden, Laserfasern oder Ähnliches handeln. So kann beispielsweise eine Elektrode, auch in Kombination mit einer Nadel insbesondere zum Unterspritzen von Gewebe, mit einer Laserfaser in einem chirurgischen Instrument kombiniert werden.

Bei dem chirurgischen Instrument kann es sich um ein Resektoskop handeln. Eine Verwendung bei anderen chirurgischen Instrumenten ist ebenso denkbar.

Vorteilhafte Ausgestaltungen und Weiterbildungen ergeben sich aus den weiteren Unteransprüchen sowie aus der Beschreibung unter Bezugnahme auf die Figuren der Zeichnung.

Gemäß einer vorteilhaften Ausführungsform kann gleichzeitig jeweils nur eines der Schlittenmodule koppelbar sein. Dadurch kann jeweils ein Instrument ausgewählt werden, während zumindest ein weiteres Instrument in dem chirurgischen Instrument gelagert werden kann und die Verwendung des anderen Instruments nicht beeinträchtigt. Die Instrumente können insbesondere parallel zueinander und/oder in Fassungen geführt sein. So kann jedes Instrument in einer Art Hülse geführt werden, wobei die Fassungen mit dem Schlittenmodul gekoppelt und mitbewegt werden können. Eine Kopplung des Schlittenmoduls an die Kinematikeinheit kann beispielsweise durch zumindest ein mechanisches oder magnetisches Verbindungsmittel erfolgen, insbesondere durch eine formschlüssige oder kraftschlüssige Verbindung zwischen der Kinematikeinheit und dem jeweiligen Schlittenmodul. Während ein Schlittenmodul mit der Kinematikeinheit gekoppelt ist sind bevorzugt alle weiteren Schlittenmodule nicht mit der Kinematikeinheit gekoppelt. Daher kann ein gewünschtes Instrument zur Anwendung mit dem chirurgischen Instrument, wie einem Resektoskop, ausgewählt werden.

Gemäß einer vorteilhaften Ausführungsform kann die Kinematikeinheit zum Verschieben der Schlittenmodule entlang einer vorbestimmten Achse ausgebildet sein. Die vorbestimmte Achse verläuft insbesondere in Längsrichtung des chirurgischen Instruments. Insbesondere ist die vorbestimmte Achse parallel zu der Führung der Instrumente ausgerichtet. Insbesondere kann sich jedes Schlittenmodul axial in Längsrichtung des chirurgischen Instruments verschieben.

Gemäß einer vorteilhaften Ausführungsform können das ersten Schlittenmodul und das zweite Schlittenmodul selektiv einzeln oder gemeinsam an der Schlittenbasis fixierbar sein. Die Schlittenbasis kann insbesondere die zumindest zwei Schlittenmodule unabhängig voneinander kontaktieren und/oder fixieren. In der Schlittenbasis können beispielsweise mehrere Ausnehmungen vorgesehen sein, wobei in jeweils einer Ausnehmung jeweils ein Schlittenmodul fixiert werden kann. Dies kann beispielsweise über Rückhaltemittel erfolgen. Des Weiteren können magnetische Bereiche an der Schlittenbasis vorgesehen sein, sodass die Rückhalteelemente an gewünschten Positionen magnetisch an der Schlittenbasis gehalten werden können.

Gemäß einer vorteilhaften Ausführungsform kann jedes Schlittenmodul daher Rückhaltemittel aufweisen, die zum Halten des jeweiligen Schlittenmoduls an der Schlittenbasis ausgebildet sind. Vorteilhafterweise kann dann jedes Schlittenmodul separat an der Schlittenbasis gehalten oder von dieser gelöst werden. Die Auswahl des jeweiligen Schlittenmoduls bzw. des jeweiligen Instruments kann über ein Betätigungselement erfolgen.

Bei dem Rückhaltemittel kann es sich um ein Rastelement, ein Ösenelement, ein Spreizelement oder ähnliches handeln. Des Weiteren kann das Rückhalteelement zumindest teilweise magnetisch ausgebildet sein, sodass es durch eine Magnetkraft, beispielsweise mittels elektromagnetischer Kopplung, an der Schlittenbasis gehalten werden kann.

Gemäß einer vorteilhaften Ausführungsform können die Rückhaltemittel eine axiale Sicherung in einer Verschiebungsrichtung des jeweiligen Schlittenmoduls bewirken. Die Verschiebungsrichtung kann dabei mit der vorbestimmten Achse übereinstimmen. Vorteilhafterweise kann jedes Schlittenmodul an einer Seitenfläche mit der Schlittenbasis kontaktiert sein und/oder an dieser fixiert sein. Wird die axiale Sicherung gelöst, können die Schlittenmodule daher unabhängig voneinander von der Schlittenbasis entfernt werden. Das Schlittenmodul mit dem nicht verwendeten Instrument kann weiterhin durch die separate axiale Sicherung an der Schlittenbasis fixiert bleiben.

Gemäß einer vorteilhaften Ausführungsform können in einem kraftfreien Ausganszustand beide Schlittenmodule an der Schlittenbasis durch das jeweilige Rückhaltemittel gehalten sein. Mit anderen Worten kann dadurch ein sogenannter verriegelte Ausgangszustand bereitgestellt werden, in welchem sich keines der Instrumente von der Schlittenbasis entfernen kann. Dadurch wird eine Absicherung bereitgestellt, wodurch eine ungewollte Verwendung der Instrumente sowie eine Beschädigung der Instrumente, insbesondere durch unkontrolliertes Herausfahren aus dem Instrumentenschaft, verhindert werden kann.

Gemäß einer vorteilhaften Ausführungsform können die Rückhaltemittel selbstrückstellend ausgebildet sein. Insbesondere kann dazu jedes Rückhaltemittel ein Federelement aufweisen. So kann beispielsweise jeweils ein Federelement in einer Führungsnut derart gelagert sein, dass es als Druckfeder gegenüber einem jeweiligen Rückhaltemittel wirkt. Durch die selbstrückstellende Wirkung kann sichergestellt werden, dass in einem unbelasteten Ausgangszustand die Schlittenmodule verriegelt an der Schlittenbasis anliegen, sodass ein unkontrolliertes Herausfahren der Instrumente aus dem chirurgischen Instrument verhindert werden kann.

Gemäß einer vorteilhaften Ausführungsform kann ein Betätigungselement zum Lösen des ersten oder des zweiten Schlittenmoduls von der Schlittenbasis vorgesehen sein. Das Betätigungselement ist insbesondere seitlich an der Schlittenvorrichtung angeordnet, sodass dieses von einer Bedienperson während der Nutzung des chirurgischen Instruments leicht erreicht werden kann. Das Betätigungselement ist bevorzugt derart angeordnet, dass es mit einem Finger erreicht werden kann, wenn eine Bedienperson das chirurgische Instrument an eine Handhabe hält.

Vorteilhafterweise weist das Betätigungselement eine Doppelfunktion auf. Das Betätigungselement kann zum einen ein Schlittenmodul von der Schlittenbasis lösen und zum anderen dasselbe Schlittenmodul an die Kinematikeinheit koppeln.

Vorteilhaft ist somit eine Funktionsintegration geschaffen. Das Betätigungselement kann dazu eine Formschlusskopplung mit dem entsprechenden Schlittenmodul ausbilden, um die Kräfte aus der Kinematikeinheit auf das Schlittenmodul zu übertragen. So kann durch das Betätigungselement ebenso eine Kraftübertagung von der Kinematikeinheit auf das Schlittenmodul und damit auf das Instrument erfolgen, sodass die Bewegung der Kinematikeinheit auf das Instrument übertragen werden kann.

Gemäß einer vorteilhaften Ausführungsform kann das Betätigungselement als mechanischer Schieber oder als elektromagnetischer Schalter ausgebildet sein. Das Betätigungselement kann daher im Sinne eines Bedienelements verstanden werden, das mechanisch bewegt oder im Sinne eines Aktuators verstanden werden, der elektromagnetisch angesteuert werden kann. Der Schieber bzw. Schalter kann jeweils mit einem der Schlittenmodule kontaktiert sein, um dieses von der Schlittenbasis zu lösen. Durch hin und her bewegen des Schiebers bzw. Schalters kann insbesondere das jeweilige Instrument ausgewählt werden. Dazu können beispielsweise Rastpositionen für den Schieber bzw. Schalter vorgesehen sein, um eine jeweilige Position auch ohne Blickkontakt einer Bedienperson auf den Schalter bzw. Schieber einfach auszuwählen.

Das Betätigungselement kann weiterhin als eine Art Exzenter oder Hebel ausgeführt sein, der insbesondere eine Dreh- oder Schwenkbewegung ausführen kann. Dabei kann der Exzenter bzw. Hebel ebenso bevorzugt mit lediglich einem Finger einer Hand angesteuert und bedient werden. Dieser kann insbesondere um bis zu 180°, oder auch bis zu 360°, verschwenkbar sein, um das erste oder das zweite Schlittenmodul von der Schlittenbasis zu lösen und mit der Kinematikeinheit zu koppeln. Insbesondere kann der Hebel bzw. Exzenter von einer ersten vertikalen Ausrichtung, zur Kopplung mit dem ersten Schlittenmodul, in eine horizontale Ausrichtung zur Lösung von beiden Schlittenmodulen, und in eine zweite vertikale Ausrichtung, zur Kopplung mit dem zweiten Schlittenmodul, gebracht werden.

Der Hebel kann einen Führungsfortsatz mit einem Vorsprung aufweisen, wobei der Vorsprung zur Kontaktierung mit dem ersten oder dem zweiten Schlittenmodul ausgebildet sein kann, insbesondere durch Rotation des Hebels. Das erste Schlittenmodul und das zweite Schlittenmodul können jeweils eine Ausnehmung aufweisen, in die der Vorsprung eingreifen kann. Dadurch kann jedes Schlittenmodul durch den Hebel selektiv mit der Kinematikeinheit gekoppelt werden.

Für den Hebel gelten im übrigen die bereits oben bzgl. des Schiebers genannten Merkmale.

In einer weiteren Ausführung kann das Betätigungselement als ferngesteuerte Bedieneinheit vorgesehen sein. Diese kann auch von einer Position beabstandet von dem chirurgischen Instrument erreicht werden.

Gemäß einer vorteilhaften Ausführungsform kann das Betätigungselement quer zu der vorbestimmten Achse verschiebbar sein. Dadurch kann verhindert werden, dass durch Bedienung des Betätigungselements eine Verschiebung des Instruments in axialer Richtung des chirurgischen Instruments ausgelöst wird. Eine derartige Bewegungsrichtung kann auch durch einen Kippschalter ausgebildet werden, der um die vorbestimmte Achse gekippt werden kann.

Gemäß einer vorteilhaften Ausführungsform kann das Betätigungselement einen Führungsfortsatz aufweisen, der in einer jeweiligen Führungsnut des ersten und zweiten Schlittenmoduls führbar ist. In der Führungsnut können ebenso die Rückhaltemittel gelagert sein, sodass das Betätigungselement, insbesondere der Führungsfortsatz, direkt jedes Rückhaltemittel kontaktieren kann. Durch Kontakt des Führungsfortsatzes mit einem Rückhaltemittel kann dieses gezielt in der Führungsnut verschoben werden, wodurch ein Lösen oder Fixieren des Rückhaltemittels an der Schlittenbasis, und ein Koppeln mit der Kinematikeinheit erreicht werden kann.

Gemäß einer vorteilhaften Ausführungsform kann die Führungsnut quer zu der vorbestimmten Achse ausgerichtet sein. Insbesondere bestimmt dabei die Ausrichtung der Führungsnut die Bewegungsrichtung des Betätigungselements.

Bevorzugt ist die Führungsnut ebenso in einem Gehäuse der Schlittenvorrichtung ausgebildet, wobei das Bedienelement außen an dem Gehäuse angeordnet sein kann, und mit dem Führungsfortsatz durch die Führungsnut hindurchgeführt ist, um so die beiden Schlittenmodule bzw. die Rückhaltemittel zu kontaktieren. Das Gehäuse kann ebenso Teil der Kinematikeinheit sein, und sich mit dem jeweiligen Schlittenmodul zumindest teilweise mit bewegen.

Gemäß einer vorteilhaften Ausführungsform kann das Betätigungselement zum Lösen der Rückhaltemittel ausgebildet sein, um eines der Schlittenmodule von der Schlittenbasis zu lösen und mit der Kinematikeinheit zu Koppeln. So kann beispielsweise durch das Betätigungselement ein Formschluss zwischen einem Rückhaltemittel und der Schlittenbasis gelöst werden.

Gemäß einer vorteilhaften Ausführungsform können die Rückhaltmittel jeweils eine Rastnase aufweisen, wobei durch Verschieben des Betätigungselements eine der Rastnasen von der Schlittenbasis gelöst wird, und die andere Rastnase mit der Schlittenbasis in Eingriff verbleibt. Durch die Rastnase kann ein derartiger Formschluss ausgebildet werden. Jede Rastnase kann durch eine Ausnehmung in der Schlittenbasis geführt werden, und an einem Hinterschnitt in der Schlittenbasis einrasten. Zum Lösen der Rastnase von dem Hinterschnitt kann das jeweilige Rückhaltemittel in der Führungsnot verschoben werden, wodurch die Rastnase innerhalb der Ausnehmung in der Schlittenbasis verschoben werden kann. Dadurch kann die Rastnase aus der Ausnehmung herausgeführt werden, wodurch das Schlittenmodul von der Schlittenbasis gelöst werden kann.

Gemäß einer vorteilhaften Ausführungsform können die Schlittenmodule in einem mehrteiligen Gehäuse gelagert sein, wobei ein erstes Gehäuseteil die Schlittenbasis und ein zweites Gehäuseteil einen Teil der Kinematikeinheit ausbildet. So kann beispielsweise eine Gehäuseseitenwand, welche am weitesten entfernt von dem distalen Ende des chirurgischen Instruments angeordnet ist, die Schlittenbasis ausbilden. Der Rest des Gehäuses, welches zusammen mit der Gehäuseseitenwand, die die Schlittenbasis ausbildet, ein Volumen vollflächig umschließen kann, kann einen Teil der Kinematikeinheit ausbilden. Das Gehäuse kann dabei die Schlittenmodule vollflächig umschließen, und beispielsweise eine Quaderform aufweisen.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: eine Seitenansicht eines chirurgischen Instruments mit einer Schlittenvorrichtung;
- Fig. 2: eine Detailansicht aus Fig. 1 in einer isometrischen Darstellung;
- Fig. 3: eine weitere Detailansicht aus Fig. 1 in einer Seitenansicht;
- Fig. 4: eine Detailansicht einer Schlittenvorrichtung;
- Fig. 5a: eine Schnittdarstellung durch eine Ausführungsform einer Schlittenvorrichtung;
- Fig. 5b: eine Schnittdarstellung durch eine weitere Ausführungsform einer Schlittenvorrichtung;
- Fig. 6: eine weitere Schnittdarstellung durch eine Ausführungsform einer Schlittenvorrichtung;
- Fig. 7: eine Detailansicht einer Schlittenvorrichtung;
- Fig. 8: zwei Detailansichten eines Betätigungselements;
- Fig. 9: eine weitere Seitenansicht einer Schlittenvorrichtung in zwei Zuständen;
- Fig. 10: eine Seitenansicht eines chirurgischen Instruments mit einer Schlittenvorrichtung;
- Fig. 11: eine Detailansicht aus Fig. 10 in einer perspektivischen Darstellung;
- Fig. 12: eine weitere Detailansicht aus Fig. 10 in einer Seitenansicht;
- Fig. 13: eine Detailansicht der Schlittenvorrichtung aus Fig. 10;
- Fig. 14: eine Schnittdarstellung durch die Ausführungsform der Schlittenvorrichtung aus Fig. 10;
- Fig. 15: eine weitere Schnittdarstellung durch die Ausführungsform der Schlittenvorrichtung aus Fig. 10;
- Fig. 16: eine Detailansicht der Schlittenvorrichtung aus Fig. 10;
- Fig. 17: eine Detailansicht eines Betätigungselements in einer möglichen Ausführungsform.

Die beiliegenden Figuren der Zeichnung sollen ein weiteres Verständnis der Ausführungsformen der Erfindung vermitteln.

Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausgeführt ist - jeweils mit denselben Bezugszeichen versehen.

### BESCHREIBUNG VON AUSFÜHRUNGSBEISPIELEN

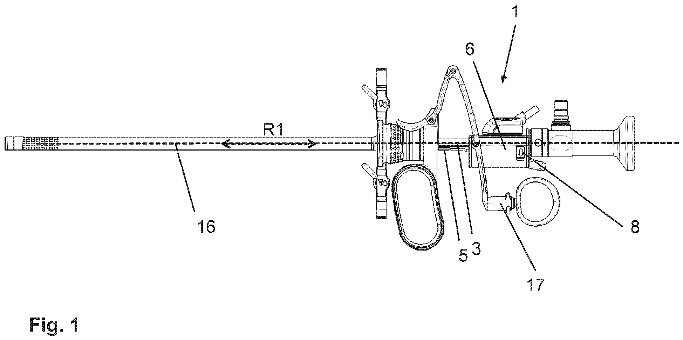

Fig. 1 zeigt eine Seitenansicht eines chirurgischen Instruments mit einer Schlittenvorrichtung 1. In dem chirurgischen Instrument sind zwei Instrumente 3, 5 angeordnet, wobei beide Instrumente an der Schlittenvorrichtung 1 gekoppelt sind. In der dargestellten Seitenansicht ist lediglich eine Außenansicht der Schlittenvorrichtung 1 erkennbar, wobei ein Gehäuse der Schlittenvorrichtung 1 einen Teil einer Kinematikeinheit 6 ausbilden kann. An der Kinematikeinheit 6 sind weiterhin Teile der Handhabungsvorrichtung, wie beispielsweise ein Daumenring 17 angeordnet. Die Schlittenvorrichtung 1 kann sich auf einer vorbestimmten Achse R1 in der Richtung der Achse R1 bewegen, wodurch ein erstes Instrument 3 oder ein zweites Instrument 5 in einem Instrumentenschaft 16 bewegt werden kann. Um das gewünschte Instrument 3, 5 auszuwählen, ist ein Betätigungselement 8 vorgesehen, das an dem Gehäuse der Schlittenvorrichtung 1 angeordnet ist.

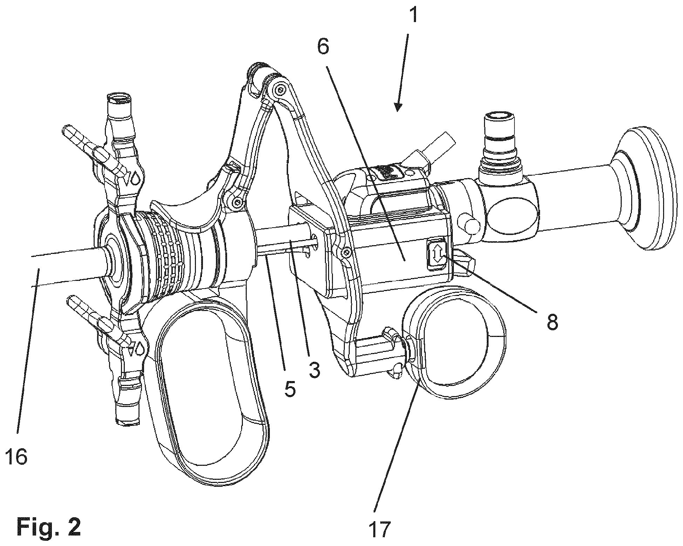

In Fig. 2 zeigt eine Detailansicht aus Fig. 1 in einer isometrischen Darstellung. Hierbei sind zum einen die Instrumente 3, 5 erkennbar, die aus dem Gehäuse der Schlittenvorrichtung 1 herausgeführt sind. Die Instrumente 3, 5 können in Fassungen geführt sein, wobei sich die Fassung insbesondere mit dem jeweiligen Instrument 3, 5 mitbewegt. Auf dem Betätigungselement 8 ist in Pfeilrichtung angezeigt, in welche Richtung sich das Betätigungselement 8 bewegen kann. In dieser Ausführungsform ist das Betätigungselement 8 als eine Art Schieber ausgebildet, und kann in der Darstellung nach unten oder nach oben bewegt werden.

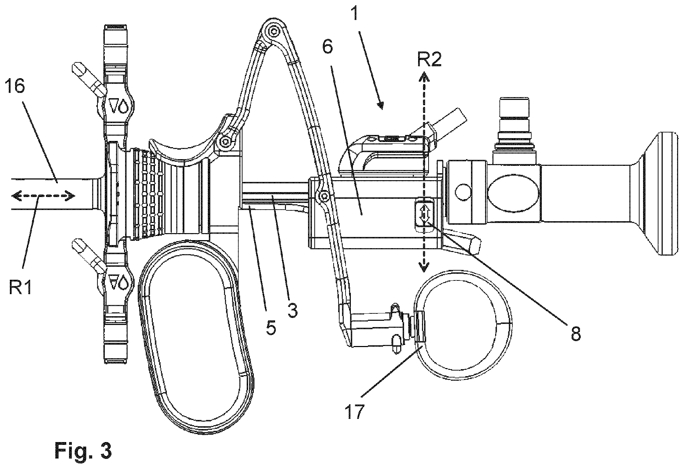

Fig. 3 zeigt eine weitere Detailansicht aus Fig. 1 in einer Seitenansicht. In dieser Ansicht ist die Bewegungsrichtung auf der Achse R2 des Schiebers dargestellt, die quer zu einer Bewegungsrichtung entlang der Achse R1 ausgerichtet ist. Durch ein Verschieben des Betätigungselements 8 kann daher ein unkontrolliertes Herausfahren eines Instruments 3, 5 aus dem Instrumentenschaft 16 verhindert werden.

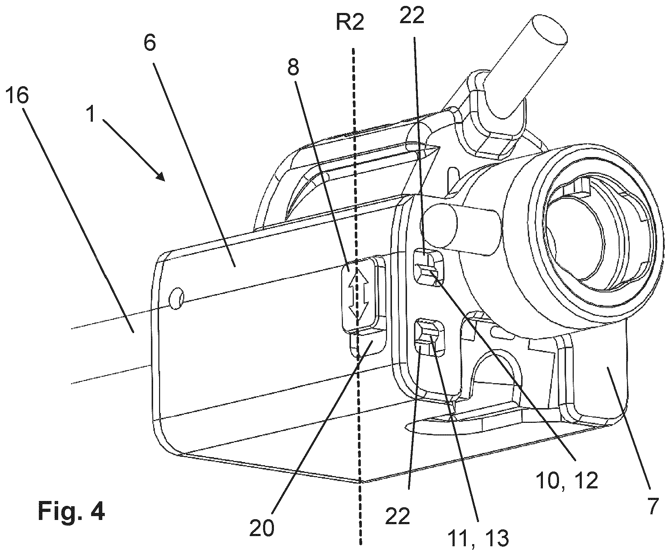

Fig. 4 zeigt eine Detailansicht einer Schlittenvorrichtung 1. Hierbei ist eine Seitenfläche der Schlittenvorrichtung 1 erkennbar, welche einem distalen Ende des chirurgischen Instruments gegenüberliegend angeordnet ist. Die Seitenfläche kann beispielsweise als Schlittenbasis 7, insbesondere ortsfeste Schlittenbasis 7, ausgebildet sein, und insbesondere eine Seitenfläche der Schlittenvorrichtung 1 ausbilden. In der Schlittenbasis 7 sind zwei Ausnehmungen 22 erkennbar, in welchen jeweils ein Rückhaltemittel 10, 11 angeordnet ist. Das Rückhaltemittel 10, 11 greift mit einer jeweiligen Rastnase 12, 13 durch die Ausnehmung 22 hindurch, und kann sich dort an einem Hinterschnitt in der Ausnehmung 22 verhaken. Dadurch kann insbesondere in einem kraftlosen Ausgangszustand ein verriegelter Zustand hergestellt werden. In einem derartigen Zustand sind beide Instrumente 3, 5 an der Schlittenbasis 7 verankert, sodass ein Herausrutschen jedes Instrument 3, 5 aus dem Instrumentenschaft 16 verhindert werden kann. Erst durch Bewegen des Betätigungselements 8 kann daher ein erstes Instrument 3 bzw. ein zweites Instrument 5 ausgewählt werden, und dieses an die Kinematikeinheit 6 gekoppelt werden. Dazu kann das Betätigungselement insbesondere auch zur Kraftübertragung von der Kinematikeinheit 6 auf das ausgewählte Schlittenmodul dienen.

Alternativ zu einer Rastnase kann das Rückhaltemittel 10, 11 auch ein insbesondere rastfreies Fixierungselement aufweisen, das beispielsweise als eine Art Öse ausgebildet ist. Weiterhin kann beispielsweise ein Spreizelement verwendet werden, das sich durch eine Aufspreizung mit der Schlittenbasis 7 verankert.

In einer nicht dargestellten Ausführungsform können die Rückhalteelemente 10, 11 auch als magnetische Rückhalteelemente ausgeführt sein, welche durch eine Magnetkraft das jeweilige Schlittenmodul 2, 4 an der Schlittenbasis 7 halten. In einer derartigen Ausführung kann die Schlittenbasis 7 auch ohne Ausnehmungen 22 ausgeführt sein, und insbesondere zumindest bereichsweise ein magnetisches Material aufweisen.

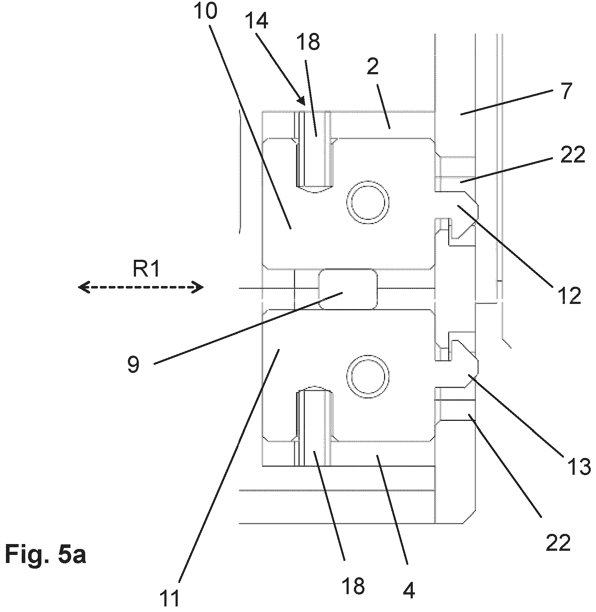

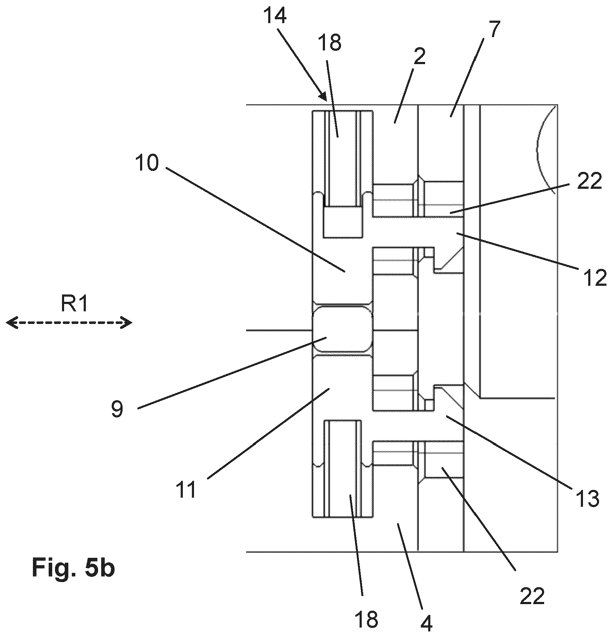

Fig. 5a und Fig. 5b zeigen je eine Schnittdarstellung durch eine Ausführungsform einer Schlittenvorrichtung 1. Die jeweilige Schnittdarstellung zeigt einen Längsschnitt durch die Schlittenvorrichtung 1, wobei lediglich ein Teilbereich dargestellt ist. In dem Teilbereich ist die Schlittenbasis 7 erkennbar, welche zwei Ausnehmungen 22 aufweist. In den Ausnehmungen 22 ist jeweils ein Rückhaltemittel 10, 11 gehalten, wobei eine jeweilige Rastnase 12, 13 jedes Rückhaltemittels 10, 11 in die Ausnehmung 22 eingreift. Dazu ist ein Hinterschnitt in der Ausnehmung 22 vorgesehen, in welcher sich die Rastnasen 12, 13 formschlüssig einhängen können. Dies dargestellte Situation zeigt einen kraftlosen Ausgangszustand, in welcher die Schlittenvorrichtung 1 verriegelt vorliegt. Die Verriegelung kann beispielsweise dadurch erreicht werden, dass die beiden Rückhaltemittel 10, 11 selbstrückstellend ausgebildet sind. In den dargestellten Ausführungsformen können dabei Federelemente 18 vorgesehen sein, wobei jeweils ein Fehlerelement 18 an ein Rückhaltemittel 10, 11 als Druckfeder wirkt. In einem kraftlosen Ausgangszustand drücken daher die Federelemente 18 gegen die Rückhaltemittel 10, 11, wobei die Rückhaltemittel 10, 11 in Richtung eines Führungsvorsatzes 9 des Betätigungselements 8 gedrückt werden. Wird der Führungsfortsatz 9 in der Darstellung nach oben oder unten bewegt, d. h. quer zu der vorbestimmten Achse R1, kann ein gewünschtes Rückhaltemittel 10 oder 11 verschoben werden. Dadurch kann sich das Rückhaltemittel 10 oder 11 von der Schlittenbasis 7 lösen, wodurch das Schlittenmodul 2 oder das Schlittenmodul 4 freigegeben wird. Dadurch kann ein gewünschtes Schlittenmodul 2 oder 4 mit der Kinematikeinheit 6 gekoppelt werden.

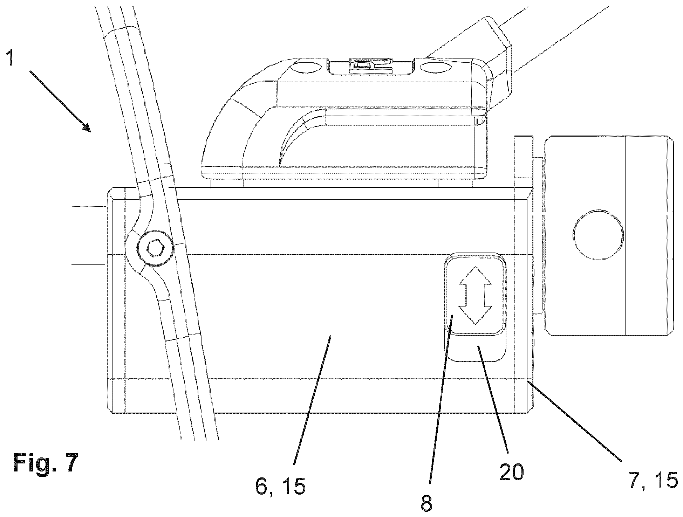

Die Federelemente 18 können in einer Führungsnut 14 geführt sein. Wie in Fig. 5b gezeigt kann ebenso der Führungsfortsatz 9 in der Führungsnut 14 geführt sein. Des Weiteren kann der Führungsfortsatz 9 versetzt zu den Federelementen 18 in einer separaten Führungsnut geführt sein, wie beispielhaft in Fig. 5a gezeigt. Die Führungsnut kann ebenso in dem Gehäuse 15, beispielsweise gezeigt in Figur 7, ausgebildet sein, sodass der Führungsfortsatz 9 von einer Außenseite des Gehäuses 15 in das Innere des Gehäuses 15 gelangen kann, um die Schlittenmodul 2, 4 bzw. die Rückhaltemittel 10, 11 zu kontaktieren bzw. zu verschieben.

In einer möglichen Ausführungsform kann daher eine Kopplung des jeweiligen Schlittenmoduls 2, 4 mit der Kinematikeinheit 6 über das Betätigungselement 8 und ein jeweiliges Haltemittel 10, 11 erfolgen, wobei diese in einer Führungsnut 14 geführt sind und entlang dieser Führungsnut 14 verschoben werden können. Andere Ausführungen, insbesondere über eine magnetische Halterung bzw. Lagerung oder ähnliches, sind ebenso denkbar.

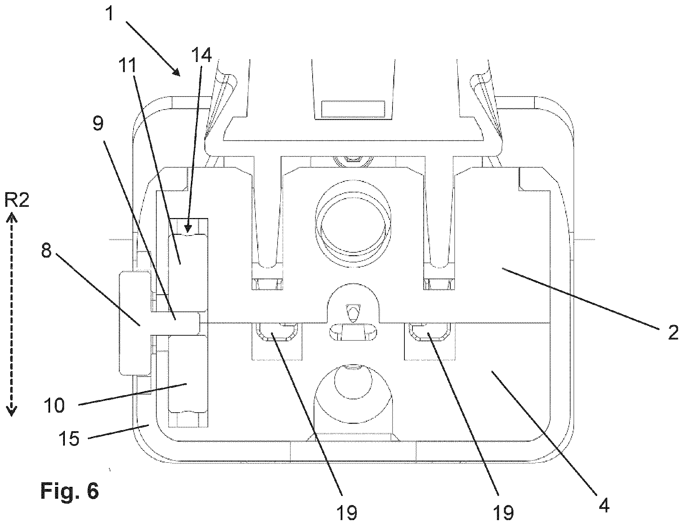

Fig. 6 zeigt eine weitere Schnittdarstellung durch eine Ausführungsform einer Schlittenvorrichtung 1. Die Schnittdarstellung zeigt einen Querschnitt, wobei dieser durch das Betätigungselement 8 verläuft. Dabei ist die Form des Betätigungselements 8 erkennbar, wobei der Führungsfortsatz 9 in das Innere des Gehäuses 15 eingreift.

Das erste Schlittenmodul 2 und das zweite Schlittenmodul 4 sind übereinander angeordnet, sodass diese separat voneinander sowie unabhängig voneinander bewegt werden können. Die Führungsnut 14 verläuft dabei teilweise in dem ersten Schlittenmodul 2 und in dem zweiten Schlittenmodul 4. Der Führungsfortsatz 9 liegt in einem kraftlosen Ausgangszustand insbesondere auf der Höhe der Kontaktflächen, an welcher sich das erste Schlittenmodul 2 und das zweite Schlittenmodul 4 kontaktieren. Dadurch kann durch eine minimale Bewegung des Betätigungselements 8 bereits eines der Schlittenmodul 2, 4 an die Kinematikeinheit 6 gekoppelt werden. Der Führungsfortsatz 9 kann daher eine formschlüssige Verbindung mit dem jeweiligen Schlittenmodul ausbilden, um Kräfte aus der Kinematikeinheit 6 auf das von der Schlittenbasis 7 entkoppelte Schlittenmodul zu übertragen.

In den Schlittenmodulen 2, 4 können weitere Ausnehmungen sowie weitere Bauteile integriert sein, wie beispielsweise ein HF-Stecker 19. Des Weiteren können Ausnehmungen vorgesehen sein, die zur Stabilisierung bei der Bewegung des jeweiligen Schlittenmoduls 2, 4 dienen.

Fig. 7 zeigt eine Detailansicht einer Schlittenvorrichtung 1. In der dargestellten Ausführungsform ist das Betätigungselement 8 als Schieber ausgeführt, wobei der Schieber innerhalb der Ausnehmung 20 nach oben geschoben dargestellt ist. Dadurch kann das in dieser Ausführungsform oben angeordnete Schlittenmodul sowie das zugehörige Instrument mit der Kinematikeinheit 6 gekoppelt werden. Die Kinematikeinheit 6 umfasst in dieser Ausführungsform einen Teil des Gehäuses 15. Ein weiterer Teil des Gehäuses 15 bildet die Schlittenbasis 7 aus. So kann ein komplettes Gehäuse 15 ausgeformt werden, das ein Volumen umschließen kann. Ein Teil des Gehäuses 15 kann sich daher mit der Kinematikeinheit 6 bewegen, wenn eines der Instrumente im Einsatz ist. Die Schlittenbasis 7 verbleibt unbeweglich in der Position, sodass das zweite Instrument, das an der Schlittenbasis 7 fixiert ist, nicht mit bewegt wird.

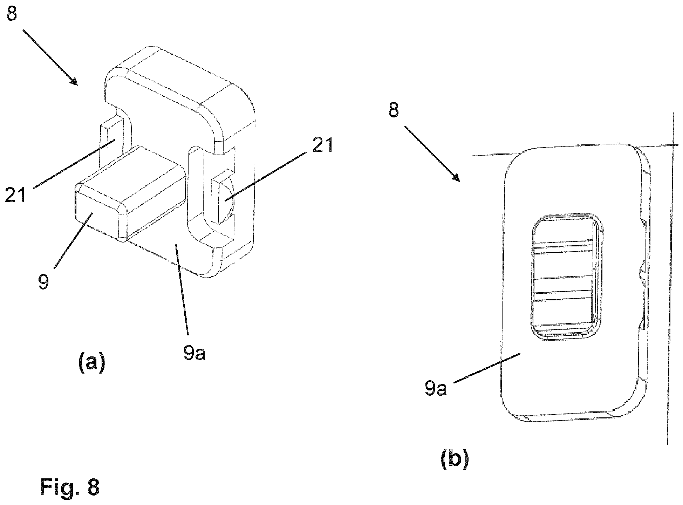

Fig. 8 zeigt zwei Detailansichten eines Betätigungselements 8. Der dargestellte Schieber weist den Führungsfortsatz 9 auf, der von einer Angriffsfläche 9a herausragend angeordnet ist. Die Angriffsfläche 9a kann von einer Bedienperson mit einem Finger erreicht werden, um das Betätigungselement 8 in die gewünschte Position zu schieben. Dazu können beispielsweise Führungen 21 vorgesehen sein, die ein sicheres Führen des Betätigungselements 8 in der Führungsnut 14 ermöglichen, gezeigt in Figur 8(a). In Figur 8(b) ist die Angriffsfläche 9a erkennbar, welche von einer Bedienperson erreicht werden kann. Diese kann eine konturierte, beispielsweise geriffelte, Oberfläche aufweisen, um eine Reibschluss mit einem Finder der Bedienperson auszubilden.

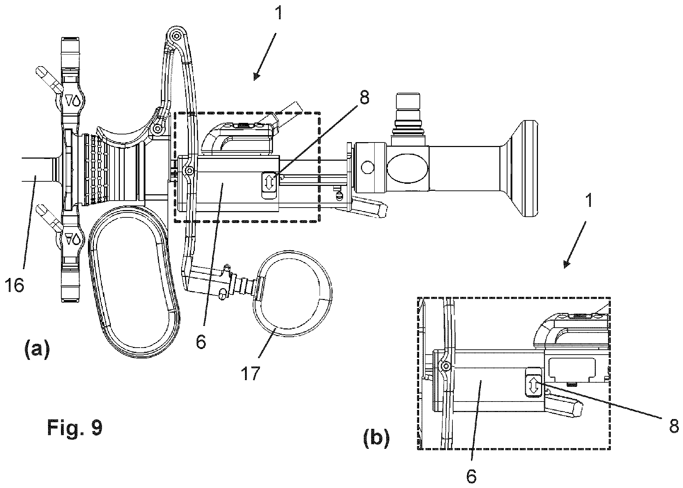

Fig. 9 zeigt eine weitere Seitenansicht einer Schlittenvorrichtung 1 in zwei Zuständen. Figur 9(a) zeigt den Zustand wie in Fig. 7, wobei das Betätigungselement 8 nach oben verschoben dargestellt ist. Folglich kann das insbesondere oben angeordnete Schlittenmodul mit der Kinematikeinheit 6 gekoppelt werden, während das zumindest eine weitere Schlittenmodul an der Schlittenbasis 7 verankert verbleibt. In Figur 9 (b) ist ein weiterer Zustand gezeigt, in welchem das Betätigungselement 8 nach unten verschoben dargestellt ist. Folglich kann ein anderes Schlittenmodul mit der Kinematikeinheit gekoppelt werden, während das zuvor gekoppelte Schlittenmodul wieder an der Schlittenbasis verankert wird.

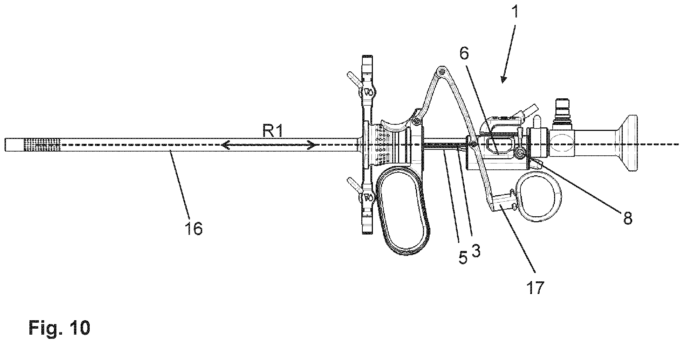

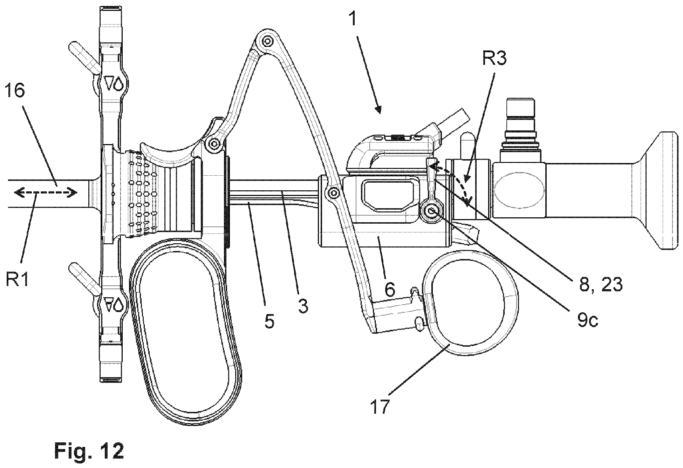

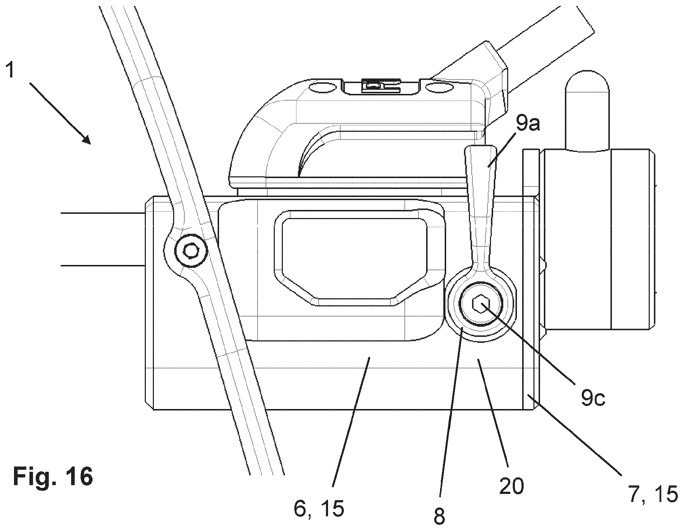

Die Figuren 10 bis 17 zeigen eine weitere mögliche Ausführungsform einer Schlittenvorrichtung 1, wobei insbesondere das Betätigungselement 8 in einer weiteren möglichen Ausführungsform gezeigt ist. In den weiteren Merkmalen kann diese Ausführungsform, mit der in den Figuren 1 bis 9 gezeigten Ausführungsform übereinstimmen, sodass nicht jedes vorangehend beschriebene Merkmal im Detail erneut wiedergegeben ist.

Fig. 10 zeigt eine Seitenansicht eines chirurgischen Instruments mit einer Schlittenvorrichtung 1 in einer derartigen weiteren Ausführungsform. Das Betätigungselement 8 ist als Hebel bzw. Exzenter ausgeführt, wobei der Hebel bzw. Exzenter um eine Achse gedreht bzw., insbesondere händisch, verschwenkt werden kann, um eines der Schlittenmodule mit der Kinematikeinheit zu koppeln. Das Betätigungselement 8 kann zum einen ein Schlittenmodul 2, 4 von der Schlittenbasis 7 lösen und zum anderen dasselbe Schlittenmodul 2, 4 an die Kinematikeinheit 6 koppeln. Die Kinematikeinheit 6 kann dabei einen Teil eines Gehäuses 15, einen Daumenring 17 oder ähnliches umfassen.

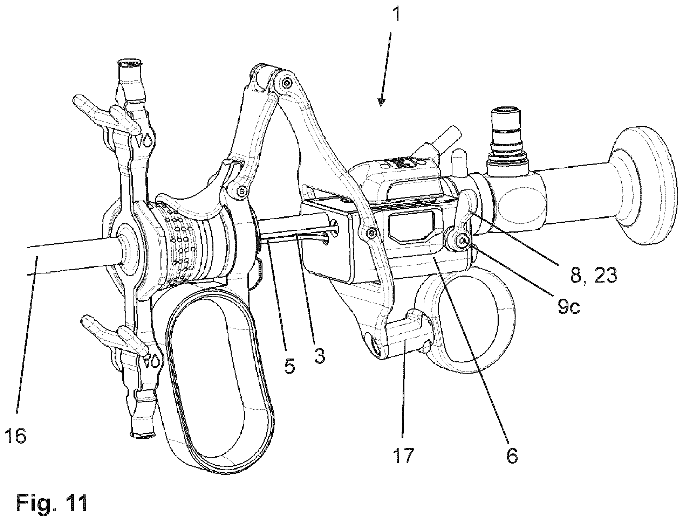

Die Figuren 11, 12 und 16 zeigen jeweils eine Detailansicht aus Fig. 10 in einer perspektivischen Darstellung bzw. einer Seitenansicht. Das Betätigungselement 8 weist in dieser Ausführungsform eine Rotationsachse 9c und ein daran angeordnetes Hebelelement 23 auf, wobei das Hebelelement 23 zumindest eine Angriffsfläche 9a ausbildet. Insbesondere sind an dem Hebelelement 23 zwei gegenüberliegende Angriffsflächen 9a vorgesehen, sodass der Hebel in entgegengesetzten Rotationsrichtungen R3 durch Druck, insbesondere händischen Druck mit einem Finger gegen das Hebelelement 23, rotiert werden kann.

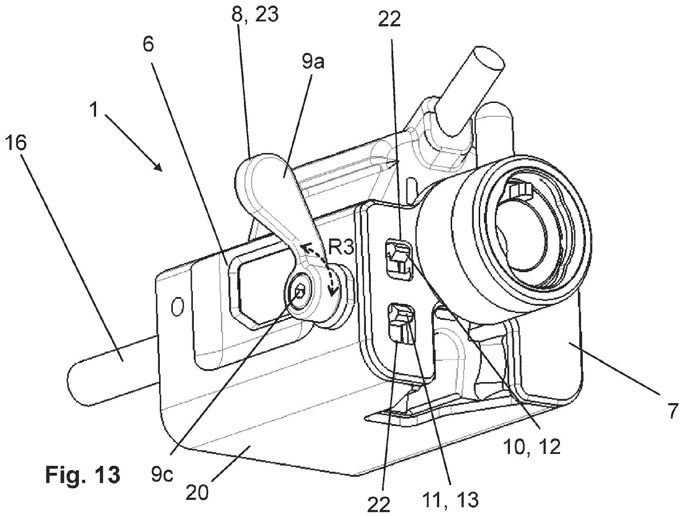

Fig. 13 zeigt eine Detailansicht der Schlittenvorrichtung 1 aus Fig. 10. Durch Rotation des Hebels um die Rotationsachse 9c kann eines der Schlittenmodule 2, 4 von der Schlittenbasis 7 gelöst werden, wobei das Hebelelement 23 dabei entlang der Rotationsrichtung R3 rotiert werden kann. So kann das Hebelelement 23 insbesondere in einem Winkel von bis zu 180° verschwenkt werden, um das erste oder das zweite Schlittenmodul 2, 4 freizugeben. Die Darstellung zweigt den Hebel bzw. das Hebelelement 23 in einer ersten vertikalen Ausrichtung zur Kopplung mit dem ersten Schlittenmodul 2. Wird der Hebel bzw. das Hebelelement 23 nach recht geschwenkt, so kann es in eine horizontale Ausrichtung zur Lösung von beiden Schlittenmodulen 2, 4 gebracht werden. Anschließend kann der Hebel bzw. das Hebelelement 23 nach unten verschwenkt werden und in eine zweite vertikale Ausrichtung zur Kopplung mit dem zweiten Schlittenmodul 4 gebracht werden.

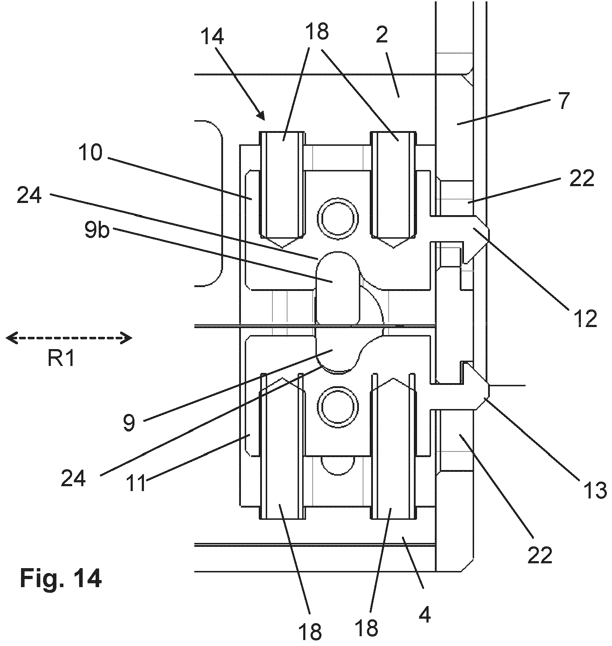

Fig. 14 zeigt eine Schnittdarstellung, wobei der Hebel bzw. das Hebelelement 23 wie in Fig. 13 vertikal ausgerichtet dargestellt ist. In einer derartigen Konfiguration wird das erste Schlittenmodul 2 von der Schlittenbasis 7 freigegeben, wobei die Rastnase 12 hierbei gelöst von der Ausnehmung 22 der Schlittenbasis dargestellt ist. Dies wird insbesondere durch einen Vorsprung 9b erreicht, der von dem Führungsabsatz 9 hervorstehend ausgeführt ist. Durch Rotation des Hebels und damit Rotation des Führungsfortsatzes 9 kann der Vorsprung 9b an eine andere Umfangsposition gebracht werden, wobei entweder das erste Schlittenmodul 2 oder das zweite Schlittenmodul 4 kontaktiert werden kann. Das erste Schlittenmodul 2 und das zweite Schlittenmodul 4 weisen dafür jeweils eine Ausnehmung 24 auf, in die der Vorsprung eingreifen kann.

Im Unterschied zu der Darstellung in den Figuren 5a und 5b sind weiterhin zwei Federelemente 18 vorgesehen. Dadurch kann vorteilhafterweise eine asymmetrische Belastung ausgeglichen werden. Eine Ausführung mit nur einem Federelement 18 ist ebenso denkbar.

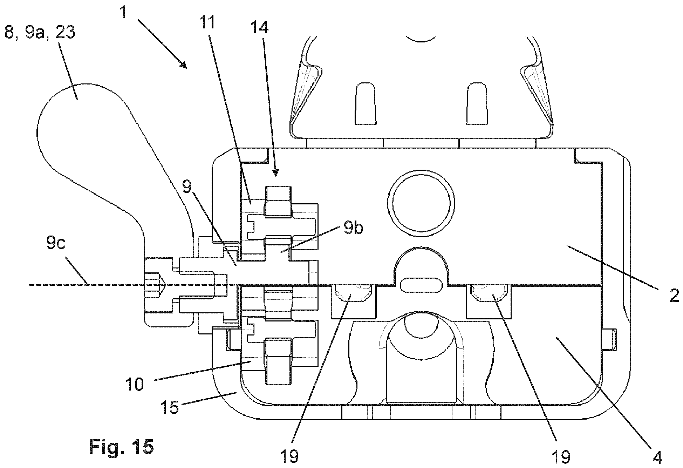

Fig. 15 zeigt eine Schnittdarstellung durch die Ausführungsform der Schlittenvorrichtung 1, wie diese in Fig. 14 gezeigt ist. Der Führungsfortsatz 9 weist in dieser Ausführungsform einen Vorsprung 9b auf. Der Vorsprung 9b ist in einem Teilbereich des Führungsfortsatzes 9 vorgesehen, um bei einer Rotationsbewegung des Führungsfortsatzes 9 eines der Schlittenmodule 2, 4 an die Kinematikeinheit 6 zu koppeln.

Der Vorsprung 9b ist insbesondere als radialer Vorsprung ausgebildet, sodass dieser durch Rotation des Hebels in unterschiedliche radiale Richtungen bezüglich des Führungsfortsatzes 9a ausgerichtet angeordnet werden kann.

Der Vorsprung 9b kann ebenso an einem Ende des Führungsfortsatzes 9 oder über die komplette Länge des Führungsfortsatzes 9 ausgebildet sein. Bevorzugt ist der Vorsprung 9b jedoch als asymmetrisches Element bzgl. einer Rotationsachse an dem Führungsfortsatz 9 vorgesehen.

Bevorzugt ist der Vorsprung 9b in die gleiche Richtung ausgerichtet wie das Hebelelement 23 bzw. die Angriffsfläche 9a des Betätigungselements 8.

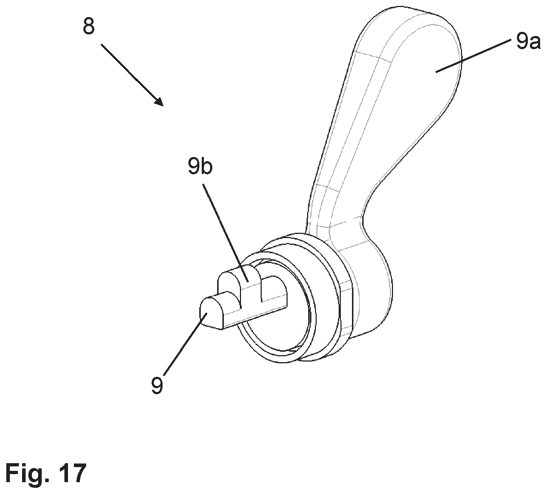

Fig. 17 zeigt eine Detailansicht eines Betätigungselements 8 in einer möglichen weiteren Ausführungsform, wie diese auch in einer Ausführung einer Schlittenvorrichtung 1 gemäß der Figuren 10 bis 16 eingesetzt werden kann.

Der Führungsfortsatz 9 sowie der Vorsprung 9b können unterschiedliche Radien aufweisen. Ebenso können der Führungsfortsatz 9 sowie der Vorsprung 9b mit dem gleichen Radius ausgeführt sein. Der Radius des Vorsprungs 9b ist insbesondere an einen Radius einer Ausnehmung 24 in der jeweiligen Schlittenbasis 2, 4 angepasst, wie in Fig. 14 gezeigt.

Obwohl die vorliegende Erfindung anhand bevorzugter Ausführungsbeispiele vorstehend vollständig beschrieben wurde, ist sie darauf nicht beschränkt, sondern auf vielfältige Art und Weise modifizierbar. Insbesondere kann die Anzahl der Instrumente und damit die Anzahl der Schlittenmodule mehr als zwei betragen. Weiterhin kann die Kinematikeinheit eine abweichende Geometrie aufweisen, insbesondere kann das Gehäuse 15 eine an die Schlittenmodule 2, 4, sowie eventuell weitere Schlittenmodule, angepasste Geometrie aufweisen. Weiterhin können die Ausführungsformen untereinander kombiniert werden, sodass weitere Ausführungsbeispiele entstehen. Insbesondere kann das Betätigungselement 8 gemäß den Figuren 10 bis 17 auch mit nur einem Federelement 18, wie in Fig. 5 gezeigt, umgesetzt werden.

### BEZUGSZEICHENLISTE

- 1: Schlittenvorrichtung
- 2: erstes Schlittenmodul
- 3: erstes Instrument
- 4: zweites Schlittenmodul
- 5: zweites Instrument
- 6: Kinematikeinheit
- 7: Schlittenbasis
- 8: Betätigungselement
- 9: Führungsfortsatz
- 9a: Angriffsfläche
- 9b: Vorsprung
- 9c: Rotationsachse
- 10: Rückhaltemittel
- 11: Rückhaltemittel
- 12: Rastnase
- 13: Rastnase
- 14: Führungsnut
- 15: Gehäuse
- 16: Schaft
- 17: Daumenring
- 18: Federelement
- 19: HF-Stecker
- 20: Ausnehmung
- 21: Führung
- 22: Ausnehmung
- 23: Hebelelement

- R1: Achse
- R2: Achse
- R3: Rotationsrichtung

## Patentansprüche

1. Schlittenvorrichtung (1) für ein chirurgisches Instrument mit zumindest zwei Instrumenten (3, 5), mit:
eine Schlittenbasis (7);
einer zur Betätigung ausgebildeten Kinematikeinheit (6);
einem ersten Schlittenmodul (2), das zur Kopplung mit einem ersten Instrument (3) ausgebildet ist; und
einem zweiten Schlittenmodul (4), das zur Kopplung mit einem zweiten Instrument (5) ausgebildet ist,
wobei die beiden Schlittenmodule (2, 4) relativ zueinander verschiebbar und einzeln mit der Kinematikeinheit (6) koppelbar sind, um das erste Instrument (3) oder das zweite Instrument (5) zu verwenden, und
wobei das ersten Schlittenmodul (2) und das zweite Schlittenmodul (4) an der Schlittenbasis (7) fixierbar sind, wenn diese nicht in Verwendung sind.

2. Schlittenvorrichtung (1) nach Anspruch 1,
**dadurch gekennzeichnet,**
**dass** gleichzeitig jeweils nur eines der Schlittenmodule (2, 4) koppelbar ist.

3. Schlittenvorrichtung (1) nach Anspruch 1 oder 2,
**dadurch gekennzeichnet,**
**dass** die Kinematikeinheit (6) zum Verschieben der Schlittenmodule (2, 4) entlang einer vorbestimmten Achse (R1) ausgebildet ist.

4. Schlittenvorrichtung (1) nach einem der vorstehenden Ansprüche,
**dadurch gekennzeichnet,**
**dass** das ersten Schlittenmodul (2) und das zweite Schlittenmodul (4) selektiv einzeln oder gemeinsam an der Schlittenbasis (7) fixierbar sind.

5. Schlittenvorrichtung (1) nach Anspruch 4,
**dadurch gekennzeichnet,**
**dass** jedes Schlittenmodul (2, 4) Rückhaltemittel (10, 11) aufweist, die zum Halten des jeweiligen Schlittenmoduls (2, 4) an der Schlittenbasis (7) ausgebildet sind.

6. Schlittenvorrichtung (1) nach Anspruch 5,
**dadurch gekennzeichnet,**
**dass** die Rückhaltemittel (10, 11) eine axiale Sicherung in einer Verschiebungsrichtung des jeweiligen Schlittenmoduls (2, 4) bewirken.

7. Schlittenvorrichtung (1) nach einem der Ansprüche 4 bis 6,
**dadurch gekennzeichnet,**
**dass** in einem kraftfreien Ausganszustand beide Schlittenmodule (2, 4) an der Schlittenbasis (7) durch das jeweilige Rückhaltemittel (10, 11) gehalten sind.

8. Schlittenvorrichtung (1) nach Anspruch 7,
**dadurch gekennzeichnet,**
**dass** die Rückhaltemittel (10, 11) selbstrückstellend ausgebildet sind, wobei insbesondere jedes Rückhaltemittel (10, 11) ein Federelement (18) aufweist.

9. Schlittenvorrichtung (1) nach einem der Ansprüche 4 bis 8,
**dadurch gekennzeichnet,**
**dass** ein Betätigungselement (8) zum Lösen des ersten oder des zweiten Schlittenmoduls (2, 4) von der Schlittenbasis (7) vorgesehen ist.

10. Schlittenvorrichtung (1) nach Anspruch 9,
**dadurch gekennzeichnet,**
**dass** das Betätigungselement (8) als mechanischer Schieber oder als elektromagnetischer Schalter ausgebildet ist.

11. Schlittenvorrichtung (1) nach Anspruch 9 oder 10,
**dadurch gekennzeichnet,**
**dass** das Betätigungselement (8) quer zu der vorbestimmten Achse (R1) verschiebbar ist.

12. Schlittenvorrichtung (1) nach einem der Ansprüche 9 bis 11,
**dadurch gekennzeichnet,**
**dass** das Betätigungselement (8) einen Führungsfortsatz (9) aufweist, der in einer jeweiligen Führungsnut (14) des ersten und zweiten Schlittenmoduls (2, 4) führbar ist.

13. Schlittenvorrichtung (1) nach Anspruch 12,
**dadurch gekennzeichnet,**
**dass** die Führungsnut (14) quer zu der vorbestimmten Achse (R1) ausgerichtet ist.

14. Schlittenvorrichtung (1) nach einem der Ansprüche 5 bis 8 und einem der Ansprüche 9 bis 13,
**dadurch gekennzeichnet,**
**dass** das Betätigungselement (8) zum Lösen der Rückhaltemittel (10, 11) ausgebildet ist, um eines der Schlittenmodule (2, 4) von der Schlittenbasis (7) zu lösen und mit der Kinematikeinheit (6) zu koppeln.

15. Schlittenvorrichtung (1) nach Anspruch 14,
**dadurch gekennzeichnet,**
**dass** die Rückhaltmittel (10, 11) jeweils eine Rastnase (12, 13) aufweisen, wobei durch Verschieben des Betätigungselements (8) eine der Rastnasen (12, 13) von der Schlittenbasis (7) gelöst wird, und die andere Rastnase (12, 13) mit der Schlittenbasis in Eingriff verbleibt.

16. Schlittenvorrichtung (1) nach einem der Ansprüche 4 bis 15,
**dadurch gekennzeichnet,**
**dass** die Schlittenmodule (2, 4) in einem mehrteiligen Gehäuse (15) gelagert sind, wobei ein erstes Gehäuseteil die Schlittenbasis (7) und ein zweites Gehäuseteil einen Teil der Kinematikeinheit (6) ausbildet.

17. Chirurgisches Instrument mit zwei Instrumenten (3, 5), aufweisend eine Schlittenvorrichtung (1) nach einem der vorstehenden Ansprüche,
**dadurch gekennzeichnet,**
**dass** die Instrumente (3, 5) über die Schlittenmodule unabhängig voneinander mit der Kinematikeinheit (6) koppelbar sind.

## Claims

1. Carriage device (1) for a surgical instrument with at least two instruments (3, 5), having:
a carriage base (7);
a kinematic unit (6) designed for actuation;
a first carriage module (2) designed for coupling to a first instrument (3); and
a second carriage module (4) designed for coupling to a second instrument (5),
wherein the two carriage modules (2, 4) are movable relative to each other and can be coupled individually to the kinematic unit (6) in order to use the first instrument (3) or the second instrument (5), and
wherein the first carriage module (2) and the second carriage module (4) can be fixed to the carriage base (7) when they are not in use.

2. Carriage device (1) according to claim 1,
**characterized**
**in that** only one of the carriage modules (2, 4) can be coupled at a time.

3. Carriage device (1) according to claim 1 or 2,
**characterized**
**in that** the kinematic unit (6) is designed to move the carriage modules (2, 4) along a predetermined axis (R1).

4. Carriage device (1) according to any of the preceding claims,
**characterized**
**in that** the first carriage module (2) and the second carriage module (4) can be selectively fixed individually or together to the carriage base (7).

5. Carriage device (1) according to claim 4,
**characterized**
**in that** each carriage module (2, 4) has retention means (10, 11) which are designed to hold the particular carriage module (2, 4) on the carriage base (7).

6. Carriage device (1) according to claim 5,
**characterized**
**in that** the retention means (10, 11) effect an axial locking in a movement direction of the particular carriage module (2, 4).

7. Carriage device (1) according to any of claims 4 to 6,
**characterized**
**in that** in a force-free initial state, both carriage modules (2, 4) are held on the carriage base (7) by the particular retention means (10, 11).

8. Carriage device (1) according to claim 7,
**characterized**
**in that** the retention means (10, 11) are designed to be self-resetting, each retention means (10, 11) in particular having a spring element (18).

9. Carriage device (1) according to any of claims 4 to 8,
**characterized**
**in that** an actuating element (8) is provided for releasing the first or the second carriage module (2, 4) from the carriage base (7).

10. Carriage device (1) according to claim 9,
**characterized**
**in that** the actuating element (8) is designed as a mechanical slide or as an electromagnetic switch.

11. Carriage device (1) according to claim 9 or 10,
**characterized**
**in that** the actuating element (8) is movable transversely to the predetermined axis (R1).

12. Carriage device (1) according to any of claims 9 to 11,
**characterized**
**in that** the actuating element (8) has a guide extension (9) which can be guided in a particular guide groove (14) of the first and second carriage module (2, 4).

13. Carriage device (1) according to claim 12,
**characterized**
**in that** the guide groove (14) is aligned transversely to the predetermined axis (R1).

14. Carriage device (1) according to any of claims 5 to 8 and any of claims 9 to 13,
**characterized**
**in that** the actuating element (8) is designed to release the retention means (10, 11) in order to release one of the carriage modules (2, 4) from the carriage base (7) and to couple it to the kinematic unit (6).

15. Carriage device (1) according to claim 14,
**characterized**
**in that** the retention means (10, 11) each have a locking lug (12, 13), one of the locking lugs (12, 13) being released from the carriage base (7) by movement of the actuating element (8), and the other locking lug (12, 13) remaining in engagement with the carriage base.

16. Carriage device (1) according to any of claims 4 to 15,
**characterized**
**in that** the carriage modules (2, 4) are mounted in a multipart housing (15), a first housing part forming the carriage base (7), and a second housing part forming part of the kinematic unit (6).

17. Surgical instrument with two instruments (3, 5), comprising a carriage device (1) according to any of the preceding claims,
**characterized**
**in that** the instruments (3, 5) can be coupled to the kinematic unit (6) independently of each other via the carriage modules.

## Revendications

1. Dispositif formant chariot (1) pour un instrument chirurgical comportant au moins deux instruments (3, 5), comportant :
une base de chariot (7) ;
une unité cinématique (6) conçue pour l'actionnement ;
un premier module de chariot (2) conçu pour être accouplé à un premier instrument (3) ; et
un second module de chariot (4) conçu pour être accouplé à un second instrument (5),
dans lequel les deux modules de chariot (2, 4) peuvent être déplacés l'un par rapport à l'autre et accouplés individuellement à l'unité cinématique (6) afin d'utiliser le premier instrument (3) ou le second instrument (5), et
dans lequel le premier module de chariot (2) et le second module de chariot (4) peuvent être fixés à la base de chariot (7) lorsqu'ils ne sont pas utilisés.

2. Dispositif formant chariot (1) selon la revendication 1,
**caractérisé en ce**
**que** respectivement un seul des modules de chariot (2, 4) peut être accouplé à la fois.

3. Dispositif formant chariot (1) selon la revendication 1 ou 2,
**caractérisé en ce**
**que** l'unité cinématique (6) est conçue pour déplacer les modules de chariot (2, 4) le long d'un axe (R1) prédéterminé.

4. Dispositif formant chariot (1) selon l'une des revendications précédentes,
**caractérisé en ce**
**que** le premier module de chariot (2) et le second module de chariot (4) peuvent être fixés sélectivement, individuellement ou conjointement sur la base de chariot (7).

5. Dispositif formant chariot (1) selon la revendication 4,
**caractérisé en ce**
**que** chaque module de chariot (2, 4) présente des moyens de retenue (10, 11) qui sont conçus pour maintenir le module de chariot (2, 4) respectif sur la base de chariot (7).

6. Dispositif formant chariot (1) selon la revendication 5,
**caractérisé en ce**
**que** les moyens de retenue (10, 11) provoquent un blocage axial dans une direction de déplacement du module de chariot (2, 4) respectif.

7. Dispositif formant chariot (1) selon l'une des revendications 4 à 6,
**caractérisé en ce**
**que**, dans un état initial sans force, les deux modules de chariot (2, 4) sont maintenus sur la base de chariot (7) au moyen du moyen de retenue (10, 11) respectif.

8. Dispositif formant chariot (1) selon la revendication 7,
**caractérisé en ce**
**que** les moyens de retenue (10, 11) sont conçus de manière à être rappelés automatiquement, dans lequel, en particulier, chaque moyen de retenue (10, 11) présente un élément élastique (18).

9. Dispositif formant chariot (1) selon l'une des revendications 4 à 8,
**caractérisé en ce**
**qu**'un élément d'actionnement (8) est prévu pour le détachement du premier ou du second module de chariot (2, 4) de la base de chariot (7).

10. Dispositif formant chariot (1) selon la revendication 9,
**caractérisé en ce**
**que** l'élément d'actionnement (8) est conçu en tant que coulisseau mécanique ou que commutateur électromagnétique.

11. Dispositif formant chariot (1) selon la revendication 9 ou 10,
**caractérisé en ce**
**que** l'élément d'actionnement (8) peut être déplacé transversalement à l'axe (R1) prédéterminé.

12. Dispositif formant chariot (1) selon l'une des revendications 9 à 11,
**caractérisé en ce**
**que** l'élément d'actionnement (8) présente un prolongement de guidage (9) qui peut être guidé dans une rainure de guidage (14) respective du premier et du second module de chariot (2, 4).

13. Dispositif formant chariot (1) selon la revendication 12,
**caractérisé en ce**
**que** la rainure de guidage (14) est orientée transversalement à l'axe (R1) prédéterminé.

14. Dispositif formant chariot (1) selon l'une des revendications 5 à 8 et l'une des revendications 9 à 13,
**caractérisé en ce**
**que** l'élément d'actionnement (8) est conçu pour libérer les moyens de retenue (10, 11) afin de détacher l'un des modules de chariot (2, 4) de la base de chariot (7) et de l'accoupler à l'unité cinématique (6).

15. Dispositif formant chariot (1) selon la revendication 14,
**caractérisé en ce**
**que** les moyens de retenue (10, 11) présentent respectivement un ergot d'encliquetage (12, 13), dans lequel, au moyen du déplacement de l'élément d'actionnement (8), l'un des ergots d'encliquetage (12, 13) est détaché de la base de chariot (7) et l'autre ergot d'encliquetage (12, 13) reste en prise avec la base de chariot.

16. Dispositif formant chariot (1) selon l'une des revendications 4 à 15,
**caractérisé en ce**
**que** les modules de chariot (2, 4) sont montés dans un boîtier (15) en plusieurs parties, dans lequel une première partie de boîtier forme la base de chariot (7) et une seconde partie de boîtier forme une partie de l'unité cinématique (6).

17. Instrument chirurgical comportant deux instruments (3, 5), présentant un dispositif formant chariot (1) selon l'une des revendications précédentes,
**caractérisé en ce**
**que** les instruments (3, 5) peuvent être accouplés à l'unité cinématique (6) indépendamment l'un de l'autre par l'intermédiaire des modules de chariot.
